Europäisches Patentamt

European Patent Office

Office européen des brevets

(11) Veröffentlichungsnummer: **0 296 506 B1**

# EUROPÄISCHE PATENTSCHRIFT

(45) Veröffentlichungstag der Patentschrift: **21.04.93**

(51) Int. Cl.5: **C12P 7/44**, //(C12N1/20, C12R1:74)

(21) Anmeldenummer: **88109721.6**

(22) Anmeldetag: **18.06.88**

(54) **Fermentative Herstellung von Dicarbonsäuren.**

(30) Priorität: **26.06.87 DE 3721119**

(43) Veröffentlichungstag der Anmeldung:
**28.12.88 Patentblatt 88/52**

(45) Bekanntmachung des Hinweises auf die
Patenterteilung:
**21.04.93 Patentblatt 93/16**

(84) Benannte Vertragsstaaten:
**AT BE CH DE ES FR GB IT LI NL**

(56) Entgegenhaltungen:
**EP-A- 0 199 972**

**CHEMICAL ABSTRACTS, Band 95, Nr. 7, September 1981, Seite 521, Zusammenfassung Nr. 113400k, Columbus, Ohio, US; & JP-A-81 26 191 (NIPPON MINING CO., LTD) 13-03-1981**

**CHEMICAL ABSTRACTS, Band 100, Nr. 11, März 1984, Seite 432, Zusammenfassung Nr. 84258j, Columbus, Ohio, US; & JP-A-58 165 795 (BIO RESEARCH CENTER CO., LTD) 30-09-1983**

**CHEMICAL ABSTRACTS, Band 105, Nr. 1, Juli 1986, Seite 500, Zusammenfassung Nr.**

**5145x, Columbus, Ohio, US; Z.T. LIU et al.: "Thermotolerant yeast producing alpha,omega-dibasic acid from n-alkane", & EUR. CONGR. BIOTECHNOL., 3rd 1984, 2, 71-6**

(73) Patentinhaber: **Henkel Kommanditgesellschaft auf Aktien**
**Postfach 1100 Henkelstrasse 67**
**W-4000 Düsseldorf-Holthausen(DE)**

(72) Erfinder: **Schindler, Joachim, Dr.**
**Am Eichelkamp 158**
**W-4010 Hilden(DE)**
Erfinder: **Hänggi, Urs, Dr.**
**Am Wasserturm 4**
**W-4000 Düsseldorf(DE)**
Erfinder: **Waldhoff, Heinrich, Dr.**
**Am Alten Broich 83 b**
**W-4018 Langenfeld(DE)**

**Beschreibung**

Die Erfindung betrifft die Umwandlung von Alkanen, Alkenen, Alkanolen, Fettsäuren und/oder deren Estern in alpha-omega-Dicarbonsäuren gleicher C-Atomzahl und gleicher Anzahl an Doppelbindungen in Gegenwart eines speziellen Mikroorganismus der Gattung Candida tropicalis. Insbesondere betrifft die Erfindung die Umwandlung von C-14-Monocarbonsäuren oder deren Estern in Tetradecandisäure (Dodecandicarbonsäure).

Dicarbonsäuren mit mehr als 10 C-Atomen sind nach Methoden der präparativen organischen Chemie im technischen Maßstab schwer herstellbar. Dies gilt insbesondere für Dicarbonsäuren, die außer den beiden endständigen Carboxylgruppen noch weitere funktionelle Gruppen wie Doppelbindungen oder Hydroxylgruppen aufweisen.

Man hat daher versucht, aus gut zugänglichen Rohstoffen mit Hilfe der Stoffwechselleistungen verschiedener Mikroorganismen Dicarbonsäuren präparativ zu erzeugen. So wird in der deutschen Patentschrift 21 40 133 vorgeschlagen, auf Alkane, primäre Alkanole oder Carbonsäuren eine Hefe der Gattung Candida und Pichia unter Fermentationsbedingungen einwirken zu lassen. Bei Einsatz von zum Beispiel einem speziellen Stamm von Candida lipolytica entstehen dabei Dicarbonsäuren, deren C-Kette gegenüber dem Ausgangsmaterial nicht oder nicht wesentlich abgebaut ist und auch ansonsten keine Veränderungen wie Einführen neuer funktioneller Gruppen aufweist. Ähnliche Verfahren, bei denen spezielle andere Mikroorganismen eingesetzt werden, sind beschrieben in den US-Patenten 3 975 234 und 4 339 536, in der britischen Patentschrift 1 450 026 sowie in den deutschen Offenlegungsschriften 21 64 626, 28 53 847, 29 37 292 und 29 51 177 und der europäischen Patentanmeldung EP-A-199 972.

Das US-Patent 4 474 882 hat ungesättigte Dicarbonsäuren zum Gegenstand. Diese werden gewonnen, indem ein Stamm der Spezies Candida tropicalis zur Umwandlung von ungesättigten Monocarbonsäuren mit 14 bis 22 C-Atomen eingesetzt wird. Die ungesättigten Dicarbonsäuren entsprechen in der Anzahl und Lage der Doppelbindung den Ausgangsmaterialien.

Nach den genannten Verfahren werden jedoch die gewünschten Dicarbonsäuren nur in recht kleinen Mengen erhalten. So sind im genannten US-Patent bei einer Fermentationsdauer von 2 Tagen Ausbeuten an Dicarbonsäure zwischen 0,65 g/l und maximal 1,96 g/l genannt. Für ein technisch genutztes Verfahren sind derartige Ausbeuten zu niedrig.

Aufgabe der Erfindung ist daher, einen Mikroorganismus bereitzustellen, der in der Lage ist, Fettsäureester, Fettsäuren und/oder Alkane, Alkene, Alkanole oder Alkenole mit 8 - 24 C-Atomen in hohen Ausbeuten in Dicarbonsäuren zu überführen und dabei insbesondere zur Herstellung von C-14-Dicarbonsäuren geeignet ist.

Gegenstand der Erfindung ist daher ein Candida tropicalis-Stamm mit der Hinterlegungsbezeichnung DSM 4131 der die Fähigkeit aufweist, Methylmyristat in einer Ausbeute von mehr als 30 g/l / 6 Tage in Tetradecandisäure umzuwandeln.

Ein weiterer Gegenstand der Erfindung ist ein Verfahren zur fermentativen Umwandlung von Fettsäureestern, Fettsäuren, Alkenen und/oder Alkanolen mit 8 - 24 C-Atomen im Carbonsäureteil in Gegenwart von Nährstoffen und gewünschtenfalls Cosubstraten in alpha-omega-Dicarbonsäuren mit gleicher C-Atom-Zahl, ohne Berücksichtigung des Alkoholteils der Ester, und gleicher Anzahl an CC-Doppelbindungen, dadurch gekennzeichnet, daß die Umwandlung in Gegenwart eines Mikroorganismenstammes Candida tropicalis DSM 4131 durchgeführt wird.

Der erfindungsgemäß eingesetzte Stamm Candida tropicalis wurde durch Mutation und Selektion aus dem öffentlich zugänglichen Stamm Candidda tropicalis ATCC 20336 gewonnen.

Als Ausgangsmaterialien können für das erfindungsgemäße Verfahren unverzweigte und verzweigte Alkane mit 8 bis 24 C-Atomen, die gewünschtenfalls 1 bis 3 innenständige konjugierte oder isolierte Doppelbindungen aufweisen, eingesetzt werden. Die Alkane können als definierte chemische Verbindungen oder als Mischung eingesetzt werden. So können beispielsweise Alkangemische eingesetzt werden wie sie aus Mineralöl erhalten werden. Weiterhin eingesetzt werden können auch Oligomerisierungsprodukte des Ethylen.

Nach einer weiteren Ausführungsform der Erfindung können auch Monocarbonsäuren eingesetzt werden. Hier sind unverzweigte Monocarbonsäuren mit endständiger Carboxylgruppe geeignet, das heißt Verbindungen, die in der Anzahl der C-Atome und Zahl und Lage der Doppelbindungen den unten aufgeführten Alkoholen entsprechen. Eine wichtige Gruppe von Monocarbonsäuren sind Fettsäuren, wie sie den natürlich vorkommenden Fettsäuretriglyceriden (Fette) zu Grunde liegen. Eingesetzt werden können Fettsäuregemische mit unterschiedlicher Zusammensetzung, das heißt sowohl destillierte, vorgereinigte Produkte wie auch die natürlich vorkommenden Mischungen. Auf diesem Gebiet stehen dem Fachmann Produkte zur Verfügung, die einen hohen oder einen niederen Grad an Unsättigung aufweisen. Beispiel für Fettsäuregemische sind die Fettsäuren, die aus Cocosöl, Palmkernöl, Palmöl, Erdnußöl, Baumwollsaatöl, Sojabohnenöl, Sonnenblumenöl, Leinöl, Rüböl, tierischen Fetten oder seetierischen Fetten herstellbar sind. Ein weiterer wichtiger Rohstoff ist die Fettsäure, die aus Ricinusöl gewonnen werden kann. Diese Fettsäure besteht zum großen Teil aus 12-Hydroxyoctadecensäure. Darüber hinaus können weitere Monocarbonsäuren mit einer sekundären Hydroxylgruppe eingesetzt werden, beispielsweise die 9- oder 10-Hydroxystearinsäure.

Eine weitere Gruppe von Monocarbonsäuren, die im erfindungsgemäßen Verfahren als Ausgangsstoffe eingesetzt werden können, sind die zu den unten aufgeführten durch Oligomerisierung von Ethylen zugänglichen Alkoholen homologen Carbonsäuren. Dabei handelt es sich größtenteils um gesättigte Carbonsäuren. Darüber hinaus können auch Monocarbonsäuren mit einer ungeraden Anzahl an C-Atomen verschiedener Provenienz eingesetzt werden.

Nach einer weiteren Variante des erfindungsgemäßen Verfahrens können auch Ester der vorgenannten Monocarbonsäuren eingesetzt werden. Dabei entstehen Dicarbonsäuren mit der gleichen Kettenlänge wie die zugrundeliegende Monocarbonsäure. Geeignet sind hier Ester mit Alkoholen der Funktionalität 1 bis 4 und bis zu 6 C-Atomen im Alkoholrest. Eine besonders bevorzugte Klasse von Estern sind die Ester der genannten Carbonsäure mit $C_1$ bis $C_4$-Alkoholen. Unter $C_1$ bis $C_4$-Alkoholen sind hier zum einen die primären Alkohole mit 1 bis 4 C-Atomen, die sekundären Alkohole mit 3 oder 4 C-Atomen, Diole mit 2 bis 4 C-Atomen, Triole mit 3 bis 4 C-Atomen oder Tetraole mit 4 C-Atomen geeignet.

Nach einer besonders bevorzugten Ausführungsform der Erfindung werden natürlich vorkommende Fettsäuretriglyceride als Ausgangsmaterial eingesetzt. Hier eignen sich die vorstehend bei den Fettsäuren diskutierten öligen oder talgartig festen Triglyceride. Bevorzugt sind flüssige Triglyceride.

Nach einer weiteren Ausführungsform der Erfindung können primäre Alkohole mit 8 bis 24 C-Atomen eingesetzt werden. Auch die Alkohole können als definierte Verbindung oder als Mischung eingesetzt werden. Zwei Gruppen von primären Alkoholen sind von besonderer Bedeutung. Zum einen die sogenannten Fettalkohole, das sind Mischungen von gesättigten und ungesättigten Alkoholen, die in ihrer Kettenlängenverteilung und Anzahl und Lage der Doppelbindungen den Fettsäureschnitten entsprechen, aus denen sie hergestellt worden sind. Fettalkohole enthalten geradzahlige Alkohole mit 1 bis 3 Doppelbindungen. Besonders wichtige Bestandteile sind im Hinblick auf das erfindungsgemäße Verfahren Fettalkohole der Kettenlänge $C_{14}$, gesättigt. Eine weitere Gruppe von primären Alkoholen sind die durch Oligomerisierung von Ethylen zugänglichen Alkohole (Ziegler-Alkohole). Dabei handelt es sich um primäre, unverzweigte, gesättigte Alkohole mit ebenfalls einer geraden Anzahl von Kohlenstoffatomen. Auch Alkohole mit ungerader Anzahl von C-Atomen verschiedener Provenienz können eingesetzt werden.

Das zur Durchführung des erfindungsgemäßen Verfahrens eingesetzte Fermentationsmedium entspricht den für die Anzucht von Candida-Arten, insbesondere von Mikroorganismen der Spezies Candida tropicalis bekannten und beschriebenen Medien. Geeignete Fermentationsmedien enthalten die üblichen Spurenelemente, eine Stickstoffquelle und eine zusätzliche, dem zu transformierenden Substrat nicht identische Kohlenstoffquelle. Unter Spurenelementen sind hier Salze der Kationen Ammonium, Kalium, Natrium, Magnesium, Calcium und Mangan mit den Anionen Phosphat, Sulfat, Chlorid zu verstehen. Als Stickstoffquelle können neben anorganischen Verbindungen wie zum Beispiel $(NH_4)_2SO_4$ auch Pepton oder Hefeextrakt eingesetzt werden.

Die Fermentationslösung enthält weiterhin als Kohlenstoffquelle ein Cosubstrat. Als Cosubstrat kann beispielsweise Natriumacetat eingesetzt werden. Weiterhin ist es möglich, daß als Cosubstrat übliche Zucker wie Glucose, Fructose, Maltose, Trehalose und dergleichen eingesetzt werden. Besonders bevorzugte Cosubstrate sind Glucose und Glycerin.

Im Hinblick auf die Verfahrensführung hat sich gezeigt, daß es günstig sein kann, wenn das Cosubstrat, also die Kohlenstoffquelle, nicht am Anfang der Fermentation sondern kontinuierlich in ausreichenden Mengen während der Umsetzung zugegeben werden. Vorteilhaft ist es auch, den pH-Wert über 7 zu halten, etwa im Bereich 7,5 - 8,5, und während der Fermentation nachzuregeln.

Nach einer weiteren Ausführungsform des erfindungsgemäßen Verfahrens ist es bevorzugt, den zur Umwandlung vorgesehenen Ausgangsverbindungen zur besseren Verteilung im Fermentationsmedium Emulgatoren zuzufügen. So werden günstige Ergebnisse erzielt, wenn insgesamt 0,1 bis 3 Gew.-%, bezogen auf Fermentationsmedium, an Emulgatoren vorhanden sind. Geeignet sind hier physiologisch verträgliche Emulgatoren und insbesonders physiologisch verträgliche nichtionische Emulgatoren. So können beispielsweise Zuckerester oder auch Sorbitanester, ethoxylierte Zuckerester, ethoxylierte Sorbitanester oder auch Alkylglycoside eingesetzt werden.

Nach dem erfindungsgemäßen Verfahren werden Dicarbonsäuren erhalten, die in ihrer Kettenlänge und in ihrem Gehalt an Doppelbindungen dem Ausgangsmaterial entsprechen, d.h. der Fachmann hat es durch Wahl des Ausgangsmaterials in der Hand, welche Dicarbonsäuren oder Dicarbonsäuremischungen er herstellen will.

Für viele Anwendungen sind die bei der Fermentation hergestellten ungesättigten Dicarbonsäuregemische direkt einsetzbar. So können die Gemische beispielsweise direkt zur Modifizierung von Polymeren, insbesondere Harzen eingesetzt werden.

**Beispiele**

Der Stamm Candida tropicalis DSM 4131 wurde wie folgt zur Synthese von Dicarbonsäuren eingesetzt: Vorkultur: Ein 500 ml Erlenmeyerkolben mit 100 ml YN-Bouillon (Fa. Difco) wurde mit der Mutante DSM 4131 beimpft und 24 Std. bei 30°C auf der Schüttelmaschine inkubiert.

Hauptkultur: Das Hauptkultur-Medium wurde mit 10 Vol% der 24 Stunden alten Vorkultur beimpft und hatte folgende Zusammensetzung: Yeast-Nitrogen-Base (Fa. Difco) + 3.0 % Glucose, 0.3 % Na-Acetat, 0.3 % Pepton und 0.5 % Hefeextrakt. Die Kultur wurde bei 30°C inkubiert und nach 24 - 30 Stunden die Transformationsphase eingeleitet, indem 50 ml Hauptkultur in neue Erlenmeyerkolben mit 50 ml Transformationsmedium übertragen wurde. Das Medium hatte folgende Zusammensetzung: Yeast-Nitrogen-Base (Fa. Difco) in 0.3 m $PO_4$-Puffer (pH 8.0) + 0.2 g MYRJ52 + 5.0g Methylmyristat oder andere Substrate. Der pH-Wert der Kulturen wurde täglich kontrolliert und auf pH 7.5 nachjustiert. Ebenso wurden täglich 0.5 % Glucose als Cosubstrat zudosiert.

Analytik: Nach 72, 96, 120 und 144 Stunden wurden Proben gezogen, die Proben mit aliquoten Mengen Acetessigester extrahiert und per Dünnschichtchromatographie oder Iatroscan-Methode analysiert. Folgende Dicarbonsäure-Ausbeuten wurden erreicht:

| Substrat: | Ausbeute: |
|---|---|
| Methyllaurat | 13g/l/144 Std. (Dodecandisäuren) |
| Methylmyristat | 44g/l/144 Std. (Tetradecandisäuren) |
| Methylpalmitat | 20g/l/144 Std. (Hexadecandisäuren) |

**Patentansprüche**

1. Candida tropicalis-Stamm mit der Hinterlegungsbezeichnung DSM 4131 der die Fähigkeit aufweist, Methylmyristat in einer Ausbeute von mehr als 30 g/1 / 6 Tage in Tetradecandisäure umzuwandeln.

2. Verfahren zur fermentiven Umwandlung von Fettsäureestern, Fettsäuren, Alkenen, Alkenolen, Alkanen und/oder Alkanolen mit 8 - 24 C-Atomen, im Falle der Fettsäureester nur bezogen auf den Carbonsäureteil, in Gegenwart von Nährstoffen und gewünschtenfalls Cosubstraten in alpha-omega-Dicarbonsäuren mit gleicher C-Atom-Zahl und gleicher Anzahl an CC-Doppelbindungen, im Falle der Fettsäureester bezogen auf den Carbonsäureteil der Ausgangssubstanz, dadurch gekennzeichnet, daß die Umwandlung in Gegenwart eines Mikroorganismenstammes Candida tropicalis DSM 4131 durchgeführt wird.

3. Verfahren nach Anspruch 2, dadurch gekennzeichnet, daß man als Ausgangssubstrat Fettsäureester mit 1 - 6 C-Atomen im Alkohol-Anteil, insbesondere Fettsäuremethylester einsetzt.

4. Verfahren nach einem der Ansprüche 2 oder 3, dadurch gekennzeichnet, daß man mit Zucker, insbesondere Glucose als Cosubstrat arbeitet, und daß das Cosubstrat während der Fermentation portionsweise zugesetzt wird.

5. Verfahren nach einem der Ansprüche 2 bis 4, dadurch gekennzeichnet, daß die Fermentation bei einem pH-Wert größer 7 durchgeführt wird, und daß der pH-Wert während der Reaktionsdauer nachgeregelt wird.

## Claims

1. A Candida tropicalis strain deposited under the code name DSM 4131 which has the ability to transform methyl myristate into tetradecanedioic acid in a yield of more than 30 g/1/6 days.

2. A process for the fermentative transformation of fatty acid esters, fatty acids, alkenes, alkanols, alkanes and/or alkanols containing 8 to 24 carbon atoms, based solely on the carboxylic acid component in the case of the fatty acid esters, in the presence of nutrients and, if desired, cosubstrates into $\alpha,\omega$-dicarboxylic acids containing the same number of carbon atoms and the same number of carbon-carbon double bonds, based on the carboxylic part of the starting substance in the case of the fatty acid esters, characterized in that the transformation is conducted in the presence of a microorganism strain Candida tropicalis DSM 4131.

3. A process as claimed in claim 2, characterized in that fatty acid esters containing 1 to 6 carbon atoms in the alcohol component, particularly fatty acid methyl ester, are used as the starting substrate.

4. A process as claimed in claim 2 or 3, characterized in that sugars, particularly glucose, are used as the cosubstrate and in that the co-substrate is added in portions during the fermentation process.

5. A process as claimed in any of claims 2 to 4, characterized in that the fermentation is carried out at a pH value above 7 and in that the pH value is readjusted during the reaction.

## Revendications

1. Souche de Candida tropicalis portant la dénomination de dépôt DSM 4131, qui présente la faculté de transformer le myristate méthylique en acide tétradécanedioïque, avec un rendement supérieur à 30 g/1/6 jours.

2. Procédé de transformation par fermentation d'esters d'acides gras, d'acides gras, d'alcènes, alcénols, alcanes et/ou alcanols comportant 8 à 24 atomes de C - dans le cas des esters d'acides gras, seulement en ce qui concerne la fraction d'acide carboxylique - en présence de substances nutritives et, le cas échéant, de co-substrats, en acides dicarboxyliques alpha-oméga possédant un nombre identique d'atomes de C et de doubles liaisons CC - dans le cas des esters d'acides gras, en ce qui concerne la fraction d'acide carboxylique de la substance de départ - caractérisé en ce que la transformation se déroule en présence d'une souche de micro-organisme Candida tropicalis DSM 4131.

3. Procédé selon la revendication 2, caractérisé en ce que l'on met en oeuvre comme substance de départ, des esters d'acides gras dont la fraction alcool comporte 1 à 6 atomes de C , en particulier des esters méthyliques d'acides gras.

4. Procédé selon l'une des revendications 2 ou 3, caractérisé en ce que l'on utilise du sucre, en particulier du glucose, comme co-substrat et en ce que ledit co-substrat est additionné par portions au cours de la fermentation.

5. Procédé selon l'une des revendications 2 à 4, caractérisé en ce que la fermentation se déroule à un pH supérieur à 7 et en ce que le pH est réajusté au cours de la réaction.